# EUROPEAN PATENT SPECIFICATION

(11) **EP 0 557 064 B1**
(45) Date of publication and mention of the grant of the patent: **11.06.1997**
(21) Application number: 93301139.7
(22) Date of filing: 17.02.1993
(51) Int. Cl.: A61K 9/16, A61K 31/485

(54) **Modified release formulation**
Arzneimittel verzögerter Wirkstoffabgabe
Composition à libération contrôlée

(30) Priority: 20.02.1992 GB 9203689; 01.05.1992 GB 9209520
(43) Date of publication of application: 25.08.1993
(73) Proprietor: Euroceltique S.A., Luxembourg (LU)
(72) Inventor: Knott, Trevor John, Wickford, Essex (GB); Leslie,Stewart Thomas, Cambridge (GB); Malkowska, Sandra Therese Antoinette, Wilburton, Ely, Cambridgeshire (GB); Miller, Ronald Brown, Dr., 4051 Basel (CH); Prater, Derek Allan, Milton, Cambrige (GB)
(74) Representative: Ruffles, Graham Keith

(56) References cited:
- EP-A- 0 214 735
- EP-A- 0 220 805
- EP-A- 0 271 193

## Description

The present invention relates to a pharmaceutical composition for oral administration and to a process for its preparation. In particular the invention relates to a pharmaceutical composition comprising uncoated spheroids comprising a water-soluble active ingredient and a spheronising agent which provides for modified release of the active ingredient.

In the pharmaceutical art water-soluble active ingredients are conventionally formulated together with insoluble excipients in an attempt to maximise their dissolution rate. One such non water soluble pharmaceutical excipient which is widely used is microcrystalline cellulose. Microcrystalline cellulose is particularly useful in the formation of spheroids by spheronisation and can itself be processed to give spheroids by simple granulation with water followed by extrusion and spheronisation. In general, the greater the proportion of microcrystalline cellulose present in a pharmaceutical composition, the easier it is to form spheroids. Microcrystalline cellulose as an excipient generally exercises little control over the release of active ingredient from a dosage form and it would therefore be expected that compositions containing a high proportion of microcrystalline cellulose would show normal release characteristics, that is fast release of active ingredient in a short period of time. Conventionally a dissolution rate of 90% or more in 45 minutes would be expected.

Film-coated spheroid preparations providing for controlled release of the active ingredient are described in EP-B-0271193. Example 4 discloses a control led release preparation comprising spheroids comprising hydromorpnone hydrochloride, microcrystalline cellulose and hydroxypropylmethylcellulose which are coated with a film coat comprising a mixture of cater soluble material (hydroxypropylmethyl cellulose). The film coating permits release of the active ingredient at a controlled rate, giving an in vitro release rate of between 12.5% and 42.5% by weight of hydromorphone after 1 hour, between 25% and 55% after 2 hours, between 45% and 75% after 4 hours and between 55% and 85% after 6 hours.

The present inventors have surprisingly found that capsules containing uncoated spheroids comprising hydromorphone hydrochloride and the spheronising agent microcrystalline cellulose exhibit modified release properties. As hydromorphone hydrochloride is highly water-soluble it would have been expected that an uncoated spheroid preparation would show normal release properties. Furthermore, on the basis of the teaching in the art, it would be expected that in order to achieve a slow release of the active ingredient a film coating would be required.

It is an object of the present invention to provide a modified release spheroid formulation comprising both a water-soluble active ingredient and a spheronising agent in which the release of the active ingredient does not depend on the presence of a release controlling film coating.

The present invention therefore provides a unit dosage form for oral administration of hydromorphone or a pharmaceutically acceptable salt thereof, characterised in that the dosage form is a capsule, sachet or cachet containing a predetermined quantity of uncoated spheroids consisting of hydromorphone or a pharmaceutically acceptable salt thereof, microcrystalline cellulose and optionally other pharmaceutically acceptable excipients, and from which spheroids the drug is released slowly to give a continuous and prolonged absorption.

The term "spheroid" is conventional in the pharmaceutical art and means a spherical granule having a diameter of between 0.1mm and 2.5mm, especially between 0.5mm and 2mm.

As used herein the term "modified release formulation" refers to formulations adapted to release the active ingredient in a slow fashion over time so as to give continuous and prolonged absorption.

Preferably the pharmaceutically acceptable salt of hydromorphone is the hydrochloride.

The composition according to the invention preferably contains from 0.1% to 25% by weight, more preferably from 0.5 to 10% by weight, especially from 1.0 to 5.0% by weight of the hydromorphone active ingredient.

The microcrystalline cellulose is a spheronising agent which may be spheronised together with the active ingredient to form spheroid cores. The microcrystalline cellulose used may be, for example, Avicel pH 101 or Avicel PH 102 (Trade Marks, FMC Corporation). Preferably the composition according to the invention contains from 10 to 99% by weight, preferably from 50 to 99% by weight of spheronising agent.

A preferred composition according to the invention comprises spheroids comprising 1.0 to 5.0% by weight hydromorphone hydrochloride and 50 to 99% by weight microcrystalline cellulose.

In addition the spheroids according to the present invention may also contain suitable quantities of other materials such as binders, colourants and flavourants that are conventional in the pharmaceutical art. Suitable binders include low viscosity water soluble polymers. Water soluble substituted cellulose ethers such as hydroxypropylmethyl cellulose are preferred in an amount of from 0.5% to 5.0% by weight.

A particularly preferred composition according to the invention comprises uncoated spheroids comprising 1 to 5% by weight hydromorphone hydrochloride, 90% to 98.5% by weight microcrystalline cellulose and 0.5 to 5% by weight hydroxypropylmethyl cellulose.

According to a further aspect the present invention provides a process for preparing a spheroid formulation according to the invention comprising
(a) blending a mixture comprising hydromorphone or a pharmaceutically acceptable salt thereof, microcrystalline cellulose and optionally other pharmaceutically acceptable excipients;
(b) extruding the blended mixture to give an extrudate;
(c) spheronising the extrudate until spheroids are formed; and
(d) drying the spheroids

Preferably the spheroids are dried until the moisture content is 7% by weight or less of the total spheroid weight as measured by Karl Fischer titration. Conveniently after drying the spheroids are sieved to give spheroids having a predetermined particle size range.

Compositions according to the present invention are formulated for oral administration in unit dosage form in conventional manner. A unit dose may consist of, for example, a capsule, sachet or cachet containing a predetermined quantity of the spheroids. It will be appreciated that the quantity is determined by the dose of active ingredient to be incorporated in a unit dose of the composition. Preferred doses will be well known to those skilled in the art.

Compositions in the form of a capsule or cachet may be administered directly via the oral route. A capsule or sachet may conveniently be sprinkled onto food to be taken as part of a meal.

In order that the invention may be well understood the following example is given by way of illustration only.

### Example 1

| | mg |
|---|---|
| Hydromorphone hydrochloride | 1.3 |
| Microcrystalline cellulose Avicel pH 101 | 77.0 |
| Hydroxypropylmethylcellulose, E15 | 1.7 |
| Purified Water | q.s. |
| Fill Weight | 80 |

The water soluble binder, hydroxypropylmethylcellulose, was included in the formulation to ensure that spheroids were produced with a low friability.

### In vitro dissolution studies

In vitro dissolution studies were conducted on compositions prepared as described in Example. The dissolution method was by Ph. Eur. paddle apparatus operated at 50rpm with distilled water at 37°C as dissolution media.

Hydromorphone hydrochloride (1.3mg), microcrystalline cellulose (77mg) and hydroxypropylmethylcellulose (1.7mg) were dry mixed. Water was added to form a granular mass which was extruded to form a uniform, free-flowing extrudate. The extrudate was spheronised and the resultant spheroids dried until they had a moisture content of about 7% by weight. The dried spheroids were sieved to obtain the 0.85-1.4mm sieve fraction.

Results are given in Table 1.

**TABLE 1**

| Time (mins) | wt% Hydromorphone released |
|---|---|
| | Example 1 |
| 10 | 52.7 |
| 20 | 69.2 |
| 30 | 73.3 |
| 45 | 77.9 |
| 60 | 85.7 |

## Claims

1. A unit dosage form for oral administration of hydromorphone or a pharmaceutically acceptable salt thereof, characterised in that the dosage form is a capsule, sachet or cachet containing a predetermined quantity of uncoated spheroids consisting of hydromorphone or a pharmaceutically acceptable salt thereof, microcrystalline cellulose and optionally other pharmaceutically acceptable excipients, and from which spheroids the drug is released slowly to give a continuous and prolonged absorption.

2. A unit dosage form according to Claim 1 wherein the hydromorphone or a pharmaceutically acceptable salt thereof is present in an amount of from 0.1% to 25% by weight.

3. A unit dosage form according to Claim 1 or Claim 2 wherein the microcrystalline cellulose is present in an amount from 10% to 99% by weight.

4. A unit dosage form according to any one of Claims 1 to 3 further comprising a pharmaceutically acceptable binder.

5. A unit dosage form according to Claim 4 wherein the binder comprises hydroxypropylmethylcellulose.

6. A unit dosage form according to any one of Claims 1-6 wherein the active ingredient is hydromorphone hydrochloride.

7. A unit dosage form according to Claim 1 comprising uncoated spheroids comprising from 1.0% to 5% by weight hydromorphone hydrochloride, from 90% to 98.5% by weight microcrystalline cellulose and from 0.5% to 5% by weight of hydroxypropylmethylcellulose.

8. A process for preparing a unit dosage form according to Claim 1 comprising the steps of:
(a) blending a mixture comprising a hydromorphone or a pharmaceutically acceptable salt thereof, microcrystalline cellulose and optionally other pharmaceutically acceptable excipients;
(b) extruding the blended mixture to give an extrudate;
(c) spheronising the extrudate until spheroids are formed;
(d) drying the speroids, and
(e) forming the speroids into a unit dosage form, characterised in that the dosage form is a capsule, sachet or cachet.

## Patentansprüche

1. Einheitsdosierungsform für orale Verabreichung von Hydromorphon oder einem pharmazeutisch verträglichen Salz davon, dadurch gekennzeichnet, daß die Dosierungsform eine Kapsel, kleiner Beutel (sachet) oder Hülle (cachet) ist, enthaltend eine vorher festgelegte Menge nicht überzogener Sphäroiden, welche aus Hydromorphon oder einem pharmazeutisch verträglichen Salz davon, mikrokristalliner Cellulose und wahlfrei anderen pharmazeutisch vertraglichen Arzneimittelträgern bestehen, und wobei von den Sphäroiden das Arzneimittel langsam unter Verursachung einer kontinuierlichen und ausgedehnten Absorption abgegeben wird.

2. Einheitsdosierungsform nach Anspruch 1, wobei das Hydromorphon oder ein pharmazeutisch verträgliches Salz davon in einer Menge von 0,1 Gew.% bis 25 Gew.% vorhanden ist.

3. Einheitsdosierungsform nach Anspruch 1 oder Anspruch 2, wobei die mikrokristalline Cellulose in einer Menge von 10 Gew.% bis 99 Gew.% vorhanden ist.

4. Einheitsdosierungsform nach einem der Ansprüche 1 bis 3, ferner umfassend ein pharmazeutisch vertragliches Bindemittel.

5. Einheitsdosierungsform nach Anspruch 4, wobei das Bindemittel Hydroxypropylmethylcellulose enthält.

6. Einheitsdosierungsform nach einem der Ansprüche 1-6, wobei der aktive Bestandteil Hydromorphonhydrochlorid ist.

7. Einheitsdosierungsform nach Anspruch 1, enthaltend nicht überzogene Sphäroiden, welche 1 Gew.% bis 5 Gew.% Hydromorphonhydrochlorid, 90 Gew.% bis 98,5 Gew.% mikrokristalline Cellulose und 0,5 Gew.% bis 5 Gew.% Hydroxypropylmethylcellulose enthalten.

8. Verfahren zum Herstellen einer Einheitsdosierungsform nach Anspruch 1, enthaltend die Stufen:
(a) Durchmischen einer Mischung, welche ein Hydromorphon oder ein pharmazeutisch verträgliches Salz davon, mikrokristalline Cellulose und wahlfrei andere pharmazeutisch verträgliche Arzneimittelträger enthält;
(b) Extrudieren der durchgemischten Mischung unter Erhalt eines Extrudats;
(c) Sphäroidbilden des Extrudats, bis Sphäroiden gebildet sind;
(d) Trocknen der Sphäroiden, und
(e) Bilden der Sphäroiden in eine Einheitsdosierungsform, dadurch gekennzeichnet, daß die Dosierungsform eine Kapsel, kleiner Beutel (sachet) oder Hülle (cachet) ist.

## Revendications

1. Formule de dosage unitaire pour administration orale d'hydromorphone ou d'un sel pharmaceutiquement acceptable de celui-ci, caractérisée en ce que la formule de dosage est une capsule, un sachet ou un cachet contenant une quantité prédéterminée de sphéroïdes non revêtus constitués d'hydromorphone ou d'un sel pharmaceutiquement acceptable de celui-ci, de cellulose microcristalline et éventuellement d'autres excipients pharmaceutiquement acceptables, sphéroïdes à partir desquels le médicament est libéré lentement pour donner une absorption continue et prolongée.

2. Formule de dosage unitaire selon la revendication 1, dans laquelle l'hydromorphone ou un sel pharmaceutiquement acceptable de celui-ci est présent en une proportion de 0,1% à 25% en poids.

3. Formule de dosage unitaire selon la revendication 1 ou la revendication 2, dans laquelle la cellulose microcristalline est présente en une proportion de 10% à 99% en poids.

4. Formule de dosage unitaire selon l'une quelconque des revendications 1 à 3, comprenant en outre un liant pharmaceutiquement acceptable.

5. Formule de dosage unitaire selon la revendication 4, dans laquelle le liant comprend de l'hydroxypropylméthylcellulose.

6. Formule de dosage unitaire selon l'une quelconque des revendications 1 à 6, dans laquelle l'ingrédient actif est le chlorhydrate d'hydromorphone.

7. Formule de dosage unitaire selon la revendication 1, comprenant des sphéroïdes non revêtus comprenant de 1,0% à 5% en poids de chlorhydrate d'hydromorphone, de 90% à 98,5% en poids de cellulose microcristalline et de 0,5% à 5% en poids d'hydroxypropylméthylcellulose.

8. Procédé pour préparer une formule de dosage unitaire selon la revendication 1, comprenant les étapes consistant à:
(a) mélanger un mélange comprenant de l'hydromorphone ou un sel pharmaceutiquement acceptable de celui-ci, de la cellulose microcristalline et éventuellement d'autres excipients pharmaceutiquement acceptables;
(b) extruder le mélange mélangé pour donner un extrudat;
(c) sphéroniser l'extrudat jusqu'à ce que des sphéroïdes se forment;
(d) sécher les sphéroïdes, et
(e) façonner les sphéroïdes en une formule de dosage unitaire, caractérisée en ce que la formule de dosage est une capsule, un sachet ou un cachet.
